# EUROPEAN PATENT APPLICATION

(11) **EP 3 392 660 A1**
(43) Date of publication of application: **24.10.2018**
(21) Application number: 16874725.1
(22) Date of filing: 29.11.2016
(51) Int. Cl.: G01N 35/04

(54) **ARRAY-TYPE FECES OCCULT BLOOD DETECTION ANALYZER**

(30) Priority: 17.12.2015 CN 201510947169
(71) Applicant: W.H.P.M. Bioresearch and Technology Co., Ltd., Beijing 100018 (CN)
(72) Inventor: WAN, John, Beijing 100018 (CN); LI, Zejiang, Beijing 100018 (CN)
(74) Representative: Roberts, Michael Austin
(86) International application number: PCT/CN2016/107600
(87) International publication number: WO 2017/101668

(57) **Abstract**

The present invention provides an array-type fecal occult blood detection analyzer. The array-type fecal occult blood detection analyzer comprises: a sample box frame (1) for carrying multiple sample boxes (11), the sample box frame (1) is an array-type structure; a conveying unit (2) for conveying the sample box frame (1), the conveying unit (2) comprises: a linear track (21) for supporting the sample box frame (1), and a driving device (23) for driving the sample box frame (1) so as to realize reciprocating linear movement; a downwards-pressing device (5) for downwards pressing the sample boxes (11); and an information collection device (4) for collecting color ribbon information presented on the test strip in the sample box (11) and label information on the sample box (11). The analyzer utilizes the linear track (21) for conveying the sample box frame (1), the feeding channel and the discharging channel are the same one, such that one channel can be cancelled, and the volume and the space of fecal occult blood detection analyzer are reduced, all sample boxes (11) are located in the same sample box frame (1), accordingly batch feeding and batch recovery of sample boxes (11) are realized.

## Description

The present invention claims a priority of Chinese patent application CN201510947169.2.

### Technical field of invention

The present invention relates to the field of medical detection, and particularly to an array-type fecal occult blood detection analyzer.

### Background of invention

"FOB": the abbreviation of English is fecal occult blood, refers to occult blood in feces, be called fecal occult blood for short, which is slight hemorrhage of digestive tract. Generally, fecal occult blood does not cause a change in feces color. Erythrocytes are damaged by digestion and there is no abnormal change in feces appearance. Bleeding cannot be confirmed visibly and microscopically. A small amount of blood cells in feces can be discovered only when the feces are tested. A small amount of bleeding for a long time is usually accompanied by anaemic symptom. Since fecal occult blood cannot be discovered directly through eyes, most patients suffering from malignant tumors of digestive tract at early stages cannot be diagnosed in time and fail to receive early intervening treatment, thus missing the best time for treatment, and fecal occult blood ambushes in people's bodies like a silent killer.

CN103543285A discloses an automatic fecal occult blood detection device for detecting a sample box. The sample box comprises a transparent sleeve, and a feces collector and test strips provided in the transparent sleeve. The detection device comprises: a import channel and a export channel are provided in coordination and respectively configured to independently operate for conveying a sample box; a transferring platform is located between the import channel and the export channel; a push rod is configured to push the sample box on the import channel to the export channel by the transferring platform, and an image acquisition device is provided in one side of export channel, wherein the image acquisition device is configured to acquire color ribbon information presented on the test strips.

The automatic fecal occult blood detection device has the following problems: the import channel and the export channel are provided in coordination, which increases a floor space of device.

### Summary of invention

The present invention provides an array-type fecal occult blood detection analyzer to solve a problem that the import channel and the export channel of fecal occult blood detection analyzer are provided in coordination, which causes a large volume of device.

For this purpose, the present invention provides an array-type fecal occult blood detection analyzer. The array-type fecal occult blood detection analyzer comprises:
a sample box frame for carrying multiple sample boxes, the sample box frame is an array-type structure;
a conveying unit for conveying the sample box frame, the conveying unit comprises: a linear track for supporting the sample box frame, and a driving device for driving the sample box frame so as to realize reciprocating linear movement;
a downwards-pressing device for downwards pressing the sample boxes; and
an information collection device for collecting color ribbon information presented on the test strip in the sample box and label information on the sample box.

Further, the sample box frame is fixed in the sample box frame mounting platform, the sample box frame mounting platform is supported in the linear track by bearing;

Further, the driving device is a first motor, the conveying unit further comprises: a screw mandrel is connected to the first motor, the sample box frame mounting platform is sleeve jointed to the screw mandrel by screw thread.

Further, the sample box frame is rectangular matrix structure, multi row and multi column through holes are provided in the sample box frame for accommodating the sample box, elastic clamping groove is provided in the top of each sample box;

The array-type fecal occult blood detection analyzer further comprises: a sample box landing unit for raising all sample boxes in one horizontal rank of sample box frame;

The sample box landing unit comprises:
Multiple bottle jacking-up rods are arranged in a row, the arrangement direction of bottle jacking-up rods and the horizontal direction of sample box frame are same. The number of bottle jacking-up rods and the number of horizontal sample boxes in the sample box frame. The bottle jacking-up rods can move up and down, and are provided below the through holes of sample box frame;
Bottle lifting rod can move up and down along the horizontal direction of sample box frame, and is provided above the through holes of sample box frame. The length of bottle lifting rod is larger than the horizontal length of sample box frame, and the bottle lifting rod can extend into a row of elastic clamping grooves and is clamped by the elastic clamping grooves;
Bottle removing rod can move up and down along the horizontal direction of sample box frame, and is provided above the through holes of sample box frame. The elastic clamping grooves can be separated from the bottle lifting rod by downwards-pressing the bottle removing rod.

Further, the array-type fecal occult blood detection analyzer further comprises: a locking device is used for locking the position of sample box frame, the locking device is connected to the second motor.

Further, the locking device comprises: an eccentric shaft, a mandril flange is pressed by the spring below the eccentric shaft, and a mandril is connected to the mandril flange. The mandril flange is provided on the side of mandril. A locking groove is provided in the sample box frame, the mandril can reciprocally move up and down through the locking groove.

Further, the locking device further comprises: a first sensor k is used for detecting the rotation position of eccentric shaft, the first sensor k is connected the second motor.

Further, the sample box frame is fixed above the sample box frame mounting platform, and the sample box frame is clamped to the sample box frame mounting platform. A guiding rod is provided on the top surface of sample box frame mounting platform, a guiding groove is provided on the bottom of sample box frame, the guiding rod is positioned in the guiding groove. A second sensor A is provided in the sample box frame mounting platform for detecting whether the sample box frame is fixed in place.

Further, the downwards-pressing device comprises: a downwards-pressing head is connected to the screw mandrel, and the downwards-pressing head is connected to the third motor.

Further, the downwards-pressing device further comprises: a crank is connected to the screw mandrel, the downwards-pressing head is connected to the screw mandrel by the crank. A rectangular hole is provided in the crank, the top of downwards-pressing head is connected into the rectangular hole. The rectangular hole limits the top of downwards-pressing head in vertical direction. When the crank moving in circular movement, the downwards-pressing head is limited in vertical direction so as to move up and down.

Further, the information collection device comprises: a barcode scanning device and a camera, the barcode scanning device and the camera are connected to the screw mandrel.

The present invention utilizes the linear track for conveying the sample box frame, the feeding channel and the discharging channel are the same one, such that one channel can be cancelled, and the volume and the space of fecal occult blood detection analyzer are reduced, all sample boxes are located in the same sample box frame, accordingly batch feeding and batch recovery of sample boxes are realized.

Further, a locking device is provided in the sample box frame, such that stability of sample box is ensured to the utmost during conveying the sample box. Thus device malfunctions due to the tilt of sample box can be effectively avoided.

### Detailed description of embodiments

Fig. 1 is a schematic diagram of an external three-dimensional structure of an array-type fecal occult blood detection analyzer according to present invention;
Fig. 2 is a right view of an external structure of an array-type fecal occult blood detection analyzer according to present invention;
Fig. 3 is a top view of an internal structure of an array-type fecal occult blood detection analyzer according to present invention;
Fig. 4 is a structural schematic diagram of a conveying unit of present invention;
Fig. 5 is a structural schematic diagram of sample box frame and sample box frame mounting platform assembled together according to present invention;
Fig. 6 is an A-A view of fig. 5;
Fig. 7 is an partial amplified view of Fig. 6;
Fig. 8 is a structural schematic diagram of a locking device when locked according to present invention;
Fig. 9 is a structural schematic diagram of a locking device when unlocked according to present invention;
Fig. 10 is a structural schematic diagram of a downwards-pressing device of present invention;
Fig. 11 is a structural schematic diagram of a downwards-pressing head of present invention under pressing condition;
Fig. 12 is a structural schematic diagram of a downwards-pressing head of present invention under non-pressing condition;
Fig. 13 is a structural schematic diagram of a sample box landing unit of present invention;
Fig. 14 is a structural schematic diagram of a bottle lifting rod of present invention;
Fig. 15 is a structural schematic diagram of a bottle jacking-up rod of present invention under starting status;
Fig. 16 is a structural schematic diagram of a bottle jacking-up rod of present invention under bottle jacking-up status;
Fig. 17 is a structural schematic diagram of a limiting protrusion of present invention;
Fig. 18 is a structural schematic diagram of a information collection device of present invention.

Numerals in the accompanying drawings:
1 sample box frame; 10 sample box frame mounting platform; 101 protrusion; 102 guiding rod; 103 lug; 11 sample box; 110 clamping groove; 13 locking device; 130 mandril; 131 mandril flange; 132 second motor; 133 eccentric shaft; 134 first sensor; 135 spring
2 conveying unit; 21 linear track; 22 screw mandrel; 23 first motor; 230 gear set; 24 sensor; 25 sliding bearing; 26 sensor; 27 sensor; 28 sensor; 29 sensor
3 sample box landing unit; 31 bottle lifting assembly; 311 sensor; 312 sensor; 313 eccentric linking rod; 314 fourth motor; 32 bottle jacking-up assembly; 320 bottle jacking-up rod; 33 guideway; 34 homing spring; 350 limiting protrusion; 351 stop block; 352 stop block; 361 driving wheel; 362 driven wheel; 363 linkage part; 364 flange; 37 bottle lifting rod; 38 bottle removing rod
4 information collection device; 41 sensor; 42 guideway; 43 barcode scanning device; 44 camera; 45 sensor
5 downwards-pressing device; 53 third motor; 54 crank; 540 rectangular hole; 55 downwards-pressing head
6 shell; 61 operating panel; 62 feeding inlet; 63 printing device; 64 switch; 65 power interface

### Detailed description of embodiments

The present invention will be described with reference to the accompanying drawings in order to understand the technical features, purposes, and effect of present invention more clearly.

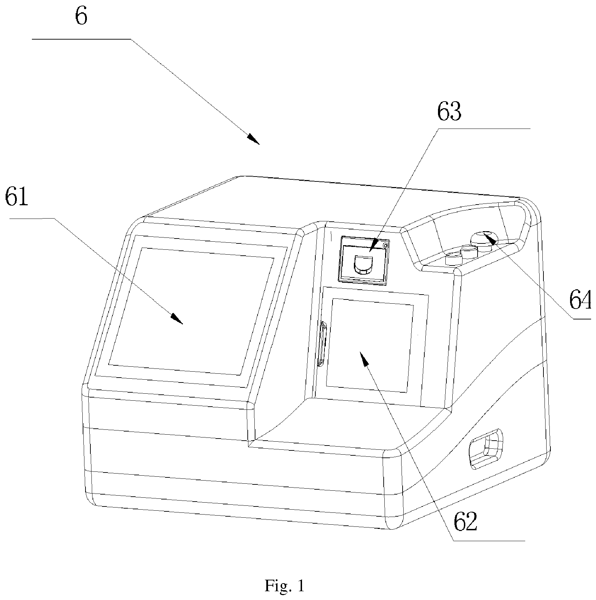

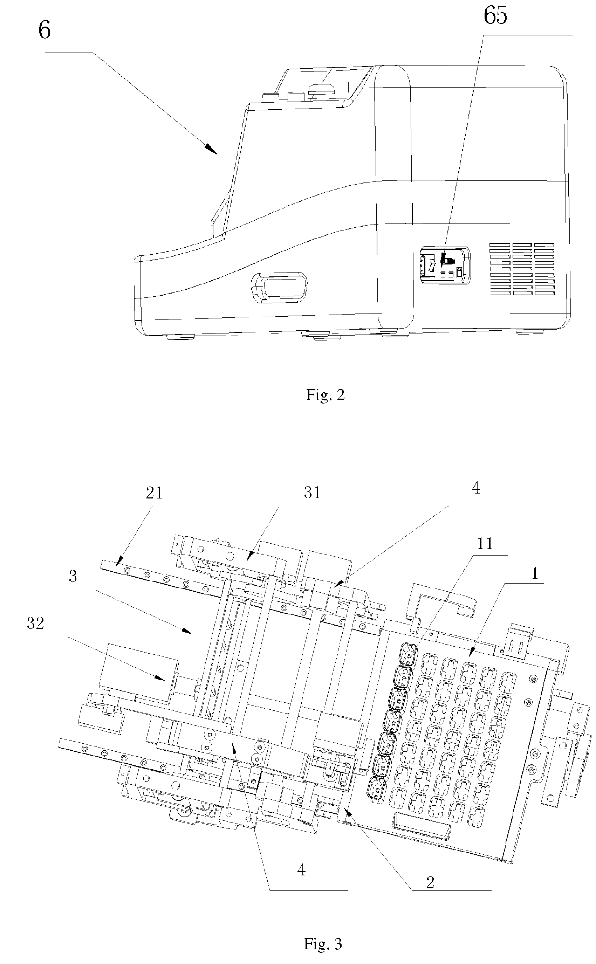

As shown in Fig. 1 and Fig. 2, an external structure of an array-type fecal occult blood detection analyzer of present invention comprises a shell 6, the shell 6 is a desk type and provided on a working surface. The shell 6 is provided with an operating panel 61 (otherwise known as controlling panel), a feeding inlet 62, a printing device 63, a switch 64 and a power interface 65. The user only need to put the sample boxes 11 in prescribed sample box frame 1, place the sample box frame 1 in the detection analyzer from the feeding inlet 62, and start the detection analyzer as required. Except starting and stopping the switch, the user operation mainly carries on the controlling panel.

As shown in Fig. 3, an internal structures of an array-type fecal occult blood detection analyzer according to present invention are provided in the shell 6, and the internal structure of an array-type fecal occult blood detection analyzer according to present invention comprises:
a sample box frame 1 for carrying multiple sample boxes 11, the sample box frame 1 is an array-type structure. With respect to the structure, pressing and detecting principle of sample box 11, please refer the prior arts, for example the Chinese utility model patent CN204613211U and the Chinese invention patent CN102879592;
a conveying unit 2 for conveying the sample box frame 1, the conveying unit is used for moving the sample box frame loaded the sample boxes in reciprocating linear way in the working area. The conveying unit 2 comprises: a linear track 21 is used for supporting the sample box frame, and a driving device is used for driving the sample box frame so as to realize reciprocating linear movement; the linear track 21 is provided in horizontal direction so as to ensure horizontal movement, non-tilt and non-leakage of sample box;
a downwards-pressing device 5 is used for downwards pressing the sample boxes 11 so as to pierce the sample box 11, then the test strips and the sample in sample box 11 are mixed so as to carry out the detection. With respect to specific pressing principle and detecting principle, please refer the prior arts;
an information collection device 4 is used for collecting color ribbon information presented on the test strip in the sample box and label information on the sample box.

The present invention utilizes the linear track for conveying the sample box frame, the sample box frame can realize reciprocating linear movement. The feeding channel and the discharging channel are the same one, such that one channel can be cancelled, and the volume and the space of fecal occult blood detection analyzer are reduced, all sample boxes are located in the same sample box frame, accordingly batch feeding and batch recovery of sample boxes are realized.

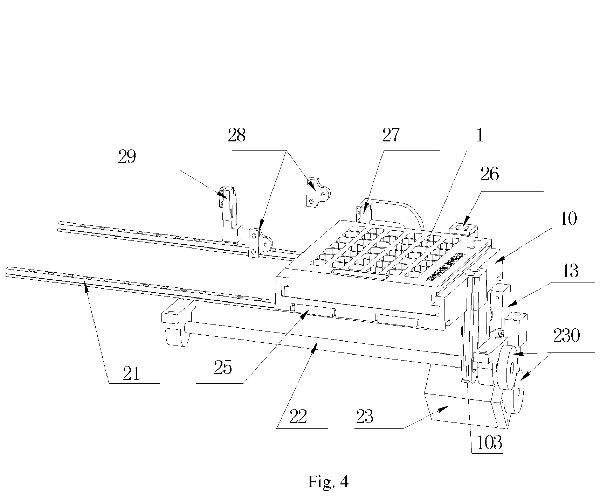

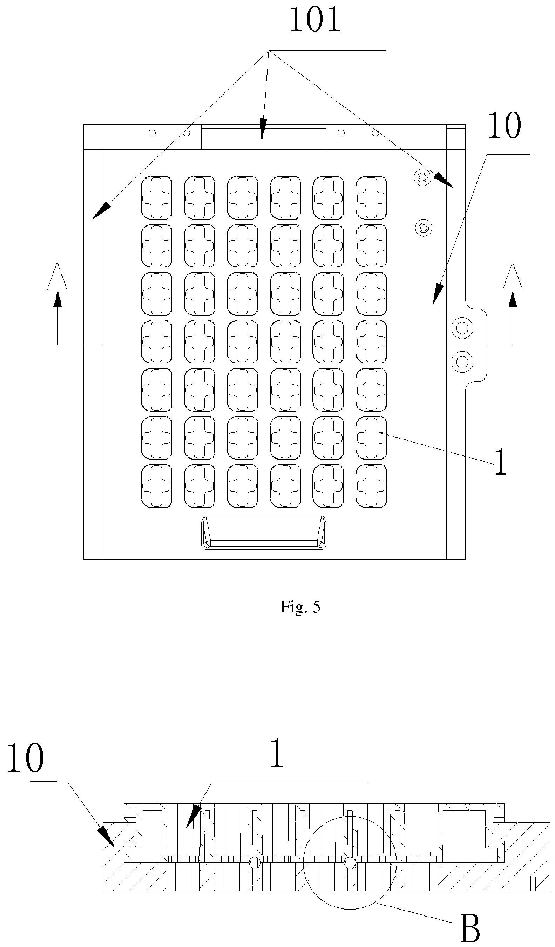

Further, as shown in Fig. 4 and Fig. 5, the sample box frame 1 is fixed in the sample box frame mounting platform 10, the sample box frame mounting platform 10 is supported in the linear track 21 by bearing (for example sliding bearing 25). The sample box frame mounting platform 10 is equivalent to a sliding block, and the sliding bearing is used for guiding the sample box frame mounting platform. Thus sliding is fast, and resistance is small; the present invention utilizes a track sliding block for conveying the sample box, the sliding block is driven by a motor, thus the movement of sliding block is more smooth, and the controlling thereof is more accurate.

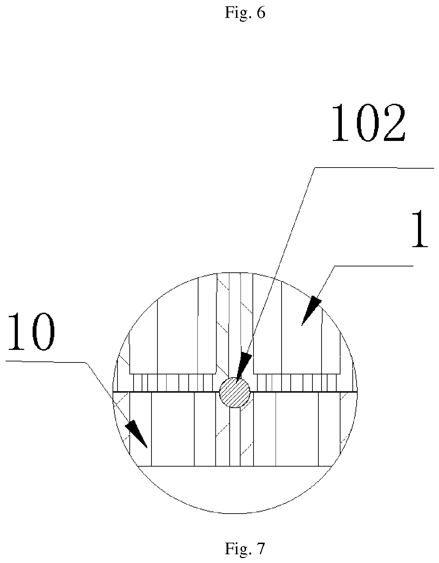

Further, as shown in Fig. 5, Fig. 6 and Fig. 7, a protrusion 101 is provided on the sample box frame mounting platform, the protrusion 101 works in with the groove structure of sample box frame so as to realize the orientation and guide of sample box frame. A guiding rod 102 is provided on the top surface of sample box frame mounting platform 10, the guiding rod 102 works in with the guiding groove on the bottom of sample box frame 1, so as to guide the sample box frame 1 when the sample box frame 1 is inserted.

As shown in Fig. 4,, the driving device is a first motor 23, the first motor 23 can rotate clockwise and counter-clockwise so as to realize reciprocating movement of sample box frame mounting platform. The conveying unit further comprises: a screw mandrel 22 is connected to the first motor 23, the sample box frame mounting platform 10 is sleeve jointed to the screw mandrel 22 by screw thread. For example, a lug 103 is prominently provided on the sample box frame mounting platform 10, the internal thread is provided in the lug 103 for matching the screw thread 22, the lug 103 is sleeve jointed to the screw mandrel 22 so as to form the feed screw nut, and the lug 103 and the screw mandrel 22 cooperatively transfer. The first motor 23 and the screw mandrel 22 are transferred by the gear set 230, thus power transmission and motion controlling are more accurate. The screw mandrel is provided in horizontal direction and is perpendicular to the linear track 21.

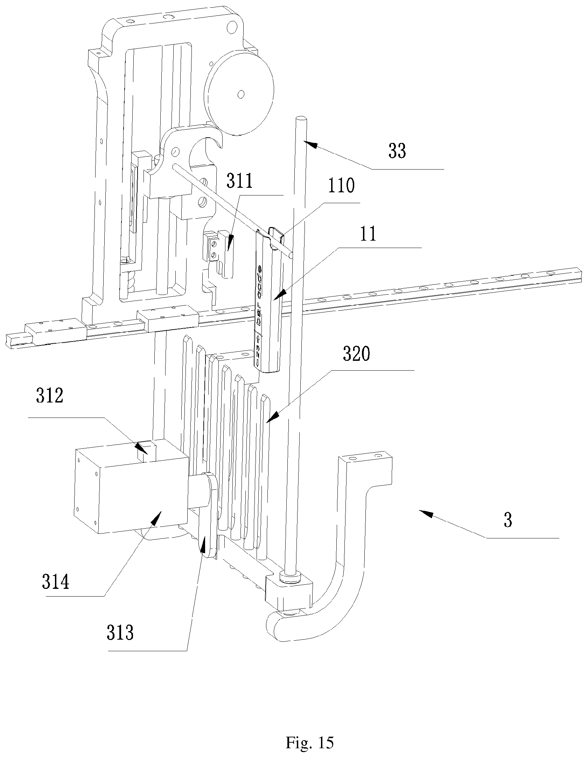

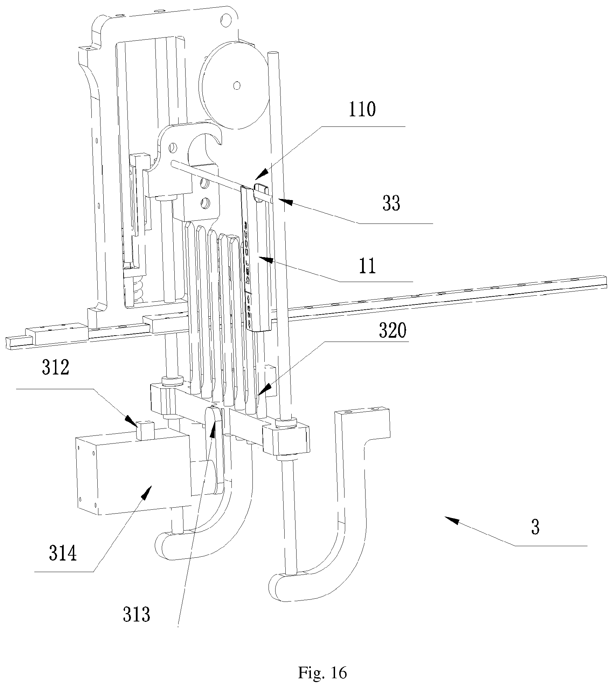

Further, as shown in Fig. 3, Fig. 4 and Fig. 5, the sample box frame 1 is rectangular matrix structure, multi row and multi column through holes are provided in the sample box frame 1 for accommodating the sample boxes, and as shown in Fig. 15 and Fig. 16, elastic clamping groove 110 is provided in the top of each sample box;

As shown in Fig. 3, Fig. 14, Fig. 15 and Fig. 16, the array-type fecal occult blood detection analyzer further comprises: a sample box landing unit 3 for raising all sample boxes in one horizontal rank of sample box frame; the sample box landing unit works in with the information collection device, thus the patient information shown in the sample box and detecting result are accurately collected in predetermined time.

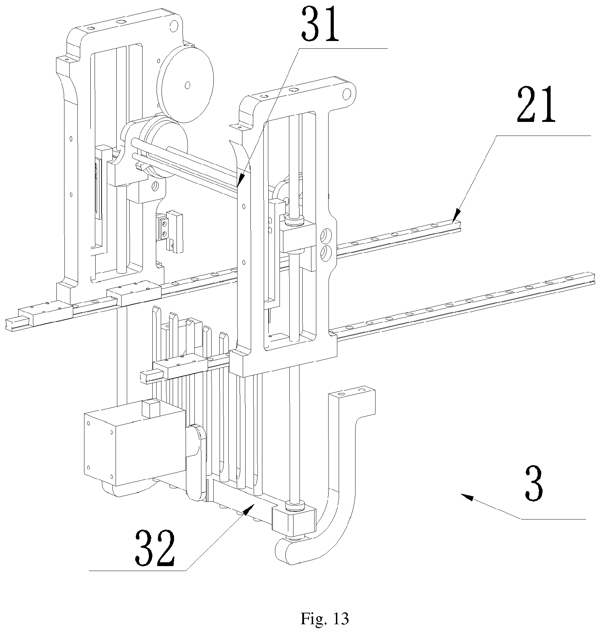

As shown in Fig. 13, the sample box landing unit 3 comprises: bottle lifting assembly 31 and bottle jacking-up assembly 32;

The bottle jacking-up assembly 32 comprises multiple bottle jacking-up rods 320 arranged in a row, the bottle jacking-up rods 320 are multiple mandrils arranged in vertical. The arrangement direction of bottle jacking-up rods 320 and the horizontal direction of sample box frame are same. The number of bottle jacking-up rods 320 and the number of horizontal sample boxes in the sample box frame are same. The bottle jacking-up rods 320 can move up and down, and are provided below the through holes of sample box frame, thus all sample boxes in one horizontal rank can be jacked up. When all sample boxes in one horizontal rank being jacked up, the bottle lifting rod 37 can extend into the elastic clamping groove on the top of sample box, thus work efficiency is increased and detection time is fast.

The bottle lifting assembly 31 comprises: bottle lifting rod 37 is a beam arranged in horizontal direction, the bottle lifting rod 37 can move up and down along the horizontal direction of sample box frame, and is provided above the through holes of sample box frame. The length of bottle lifting rod is larger than the horizontal length of sample box frame, and the bottle lifting rod 37 can extend into a row of elastic clamping groove and is clamped by the elastic clamping groove. After all sample boxes in one horizontal rank being jacked up by the bottle jacking-up rods 320, all sample boxes in one horizontal rank are clamped at the bottle lifting rod 37 from bottom to top by the elastic clamping groove 110, so as to scan the barcode by barcode scanning device and take a picture by camera, thus detection time is fast.

The sample box landing unit 3 further comprises: bottle removing rod 38 is a beam arranged in horizontal direction, the bottle removing rod 38 can move up and down along the horizontal direction of sample box frame, and is provided above the through holes of sample box frame. The elastic clamping groove 110 can be separated from the bottle lifting rod 37 by downwards-pressing the bottle removing rod, thus the sample boxes return to original place by the landing unit after collecting information and the bottles are removed from the landing unit into the sample box frame.

The bottle jacking-up assembly 31 jacks up a row of bottles from the sample box frame 1, the bottle lifting rod 37 enters into the clamping grooves of jacked sample boxes. A row of sample boxes are lifted by the landing unit, and the sample boxes return to original place by the landing unit after collecting information and the bottles are removed from the landing unit into the sample box frame.

As shown in Fig. 15, a clamping groove 110 is provided in the sample box 11 for working in with the bottle lifting rod, the diameter of bottle lifting rod 37 is larger than the width of clamping groove of sample box under natural state. A bottle jacking-up sensor H is provided below the guideway of conveying unit, that is when the sensor 312 detects the sample box 11 entering into the sample box landing unit, the fourth motor 314 starts to rotate, the eccentric linking rod 313 changes the rotation to reciprocating movement for the linking rod, the linking rod brings the bottle jacking-up rod 320 up along the guideway 33 (the guideway 33 is vertically provided and is perpendicular to the linear track 21). When the sample box 11 is jacked up until the bottle lifting rod 37 falling into the clamping groove 110 in jacked up sample box, the fourth motor 314 reversely rotates, the bottle lifting assembly 31 return to starting status.

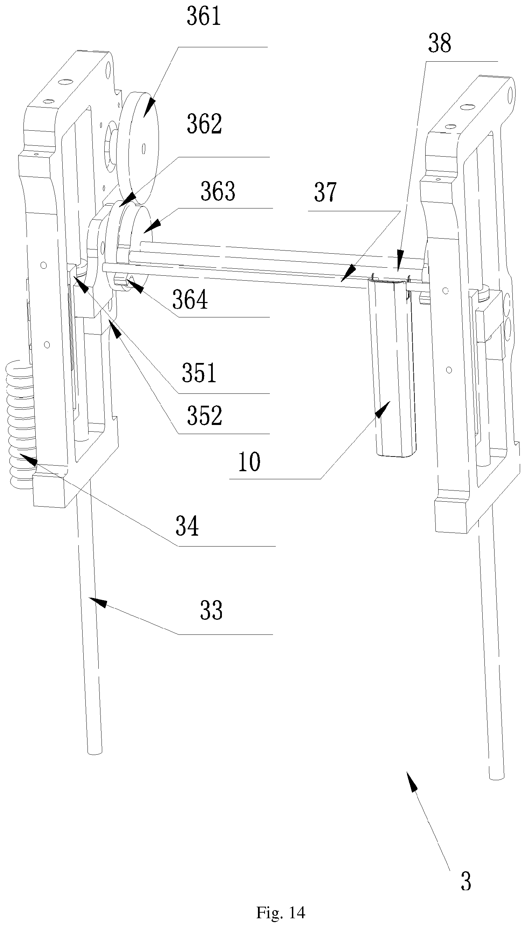

As shown in Fig. 14, the linking rod is provided with multiple bottle jacking-up rod 320, the number of bottle jacking-up rod 320 and the number of horizontal sample boxes in the sample box are same. The bottle jacking-up rod 320 is rigidly connected to the linking rod and moves with the movement of linking rod. The stop block 351 of sample box landing unit acts on limiting function for the bottle lifting rod when the bottle jacking-up rod 320 jacking up the bottles, the bottle lifting rod 37 cannot be jacked up under external force, thus the bottle lifting rod fully enters into the clamping grooves of jacked sample boxes.

The bottle lifting sensor F, namely the sensor 311 detects the sample box being jacked up, the motor (namely the fourth motor 314) drives the driving wheel 361 to rotate, the driving wheel 316 drives the driven wheel 362, the driven wheel 362 is rigidly connected a linkage part 363, the linkage part 363 is provided with a flange 364 having bottle lifting function and a bottle removing rod 38, the distance between the flange 364 having bottle lifting function and circle centre of driven wheel 362 is larger than the distance between the bottle removing rod 38 and circle centre of driven wheel 362, thus interference phenomenon of parts cannot occur during movement.

As shown in Fig. 14, the linkage part 363 synchronously rotates with the driven wheel 362. When the flange 364 having bottle lifting function rotates and contacts to the bottle lifting rod 37, the bottle lifting rod 37 with sample boxes is brought up. The bottle lifting rod is positioned above the guideway 33, and realize reciprocating linear movement on the guideway 33 or realize reciprocating linear movement across the guideway 33. When the sample box is brought up to predetermine height, the fourth motor 314 stop rotating, the fourth motor 314 reversely rotates after the information is collected, the flange having bottle lifting function and the bottle lifting rod separate from each other.

As shown in Fig. 14, a homing spring 34 is connected at the stop block 351 and the bottle lifting rod 37, the homing spring is under stretching when the sample box is brought up. When the flange having bottle lifting function is separated from the bottle lifting rod, the bottle lifting rod moves down under the action of homing spring, then the bottle lifting rod stops moving down until contacting the stop block 352, and at this moment the bottle lifting rod returns to the starting position. The linkage part 363 continues to rotate with the fourth motor 314, when the bottle removing rod contacting the sample box, the sample box is removed from the bottle lifting rod.

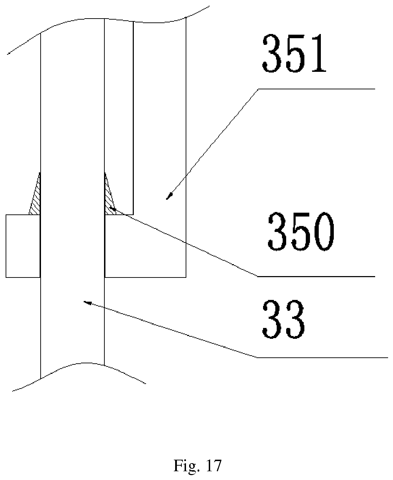

When the sample box homing sensor detecting the returned sample box, the motor (the fourth motor 314) reverses, the sample box landing unit returns to starting position. Wherein the stop block 351 is positioned above the guideway having limiting protrusion 350, as shown in Fig. 17, the limiting protrusion 350 is positioned at the position of limiting rod when the downwards-pressing rod is under the starting position. The strength of limiting protrusion 350 should be lager enough to bear the acting force when jacking the bottle, thus the position of bottle lifting rod cannot change during jacking bottle; furthermore, the strength of limiting protrusion 350 should be smaller than the tractive force when lifting the bottle lifting rod, thus the stop block can move upwards with the bottle lifting rod and cannot block the movement of bottle lifting rod during lifting the bottle; the limiting protrusion 350 preferably is inverted triangle, such shape can effectively reduce the resistance of stop block 351 when the stop block 351 moving downwards with the bottle lifting rod, such that the resistance of homing spring can be reduced.

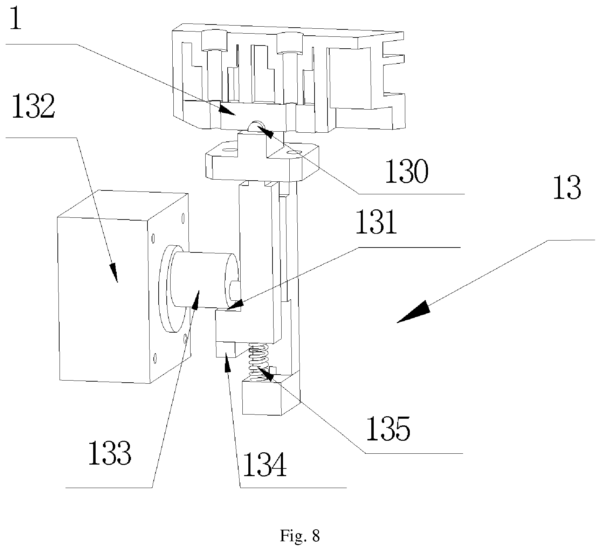

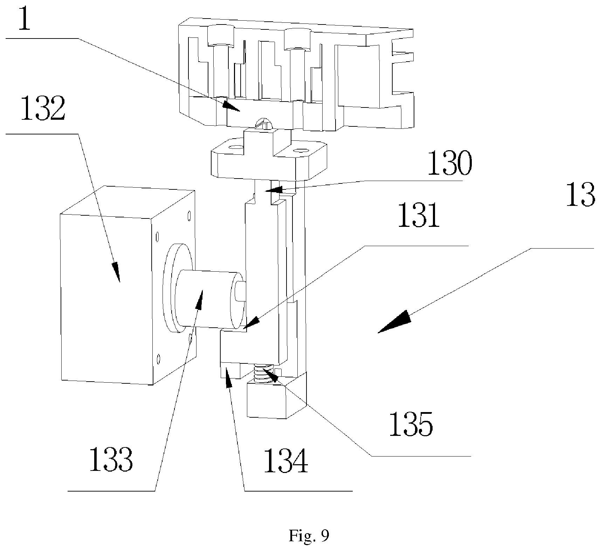

Further, as shown in Fig. 4, the array-type fecal occult blood detection analyzer further comprises: a locking device 13 is used for locking the position of sample box frame. As shown in Fig. 8 and Fig. 9, the locking device 13 is connected to the second motor 132, the locking device 13 can be locked and unlocked by the motor, such that stability of sample box is ensured during conveying the sample box, and device malfunctions due to the tilt of sample box can be effectively avoided. The locking device is positioned below the linear track 21 or the sample box frame, and locks the sample box frame from the bottom, thus interference between the locking device and the detecting parts on the top of sample box frame or other parts can be avoided.

Further, as shown in Fig. 8 and Fig. 9, the locking device 13 comprises: an eccentric shaft 133, a mandril flange 131 is pressed below the eccentric shaft by the spring 135, and a mandril 130 is connected to the mandril flange. The mandril flange 131 is positioned on the side of mandril 130. The bottom of sample box frame 1 is provided with notch or locking groove, the mandril 130 can reciprocally move up and down through the locking groove, thus locking and unlocking can be realized.

The output shaft of motor (second motor 132) can be connected to a shaft sleeve with eccentric hole or a solid eccentric shaft 133, the motor drives the shaft sleeve or eccentric shaft 133 for eccentric rotation, the lower edge of eccentric shaft acts upon the mandril flange of locking device, and works in with (mandril) spring 135, thus the mandril of locking device can reciprocally move up and down.

Further, the locking device further comprises: a first sensor k is used for detecting the rotation position of eccentric shaft, namely sensor 134, the first sensor k is connected the second motor. When the distance between the outer edge of eccentric shaft 133 and lower acting surface of mandril of locking device is positioned in minimum and maximum, the sensor K captures signal, the motor (second motor 132) stops moving. When the sample box frame is installed in place, the locking device of sample box frame can be locked, as shown in Fig. 8, the outer edge of eccentric shaft is positioned at minimum distance between the outer edge of eccentric shaft 133 and lower acting surface of mandril of locking device, the (mandril) spring 135 is under natural state, the mandril passes through the sample box frame mounting platform and enters into the sample box frame mounting platform.

The sample box frame and sample box frame mounting platform can reciprocally move together on the guideway under locking status. When the sample box frame returns to the starting position, the sample box locking device are unlocked, as shown in Fig. 9, the outer edge of eccentric shaft is positioned at maximum distance between the outer edge of eccentric shaft 133 and lower acting surface of mandril of locking device, the mandril spring is under compression state, the mandril cannot enter into the sample box frame mounting platform, and the device are unlocked.

As shown in Fig. 3, a sensor A (namely sensor 26) is provided in the sample box frame mounting platform 10 for detecting whether the sample box frame is fixed in place. If the sample box frame is not fixed in place, the sensor A can transmit the signal to information processing unit, and the detection analyzer reports errors. A sensor B (namely sensor 28) is provided in the sides of guideway (linear track 21) for detecting fixing status of sample box, that is to say this sensor is used for detecting whether the sample box is fixed in the sample box frame and mounting type of sample box is correct. If the mounting type of sample box is not correct, the sensor B can transmit the signal to information processing unit, and the detection analyzer reports errors. Furthermore, a sensor D (namely sensor 29) is provided in the sides of guideway (linear track 21) for detecting whether the motor for driving the sample box frame mounting platform is synchronized motion, such that the sample box frame mounting platform can carry out stationary motion. If the non-stationary motion exists, the sensor D can transmit the signal to information processing unit system, and the detection analyzer reports errors.

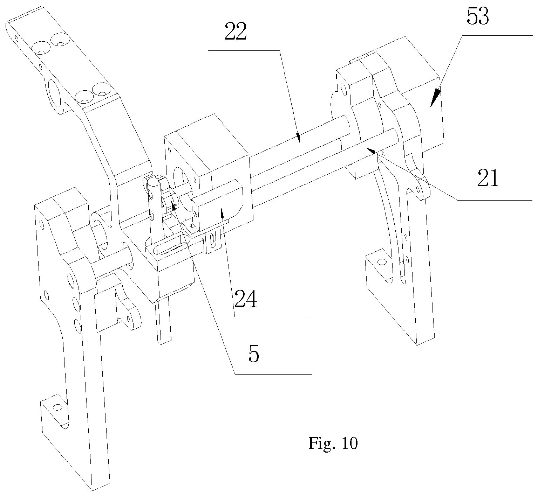

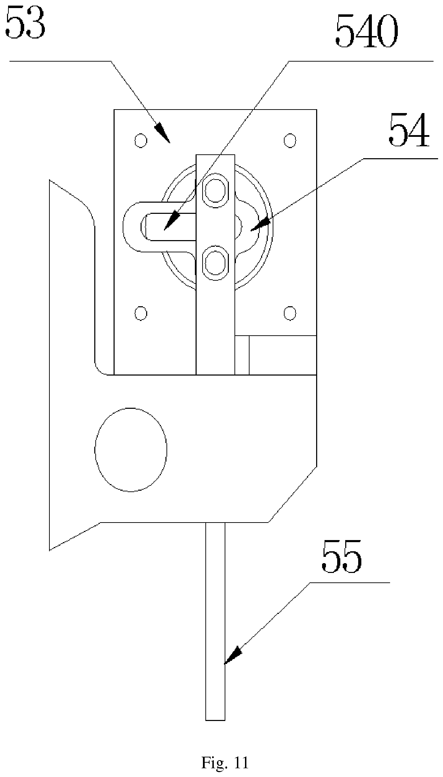

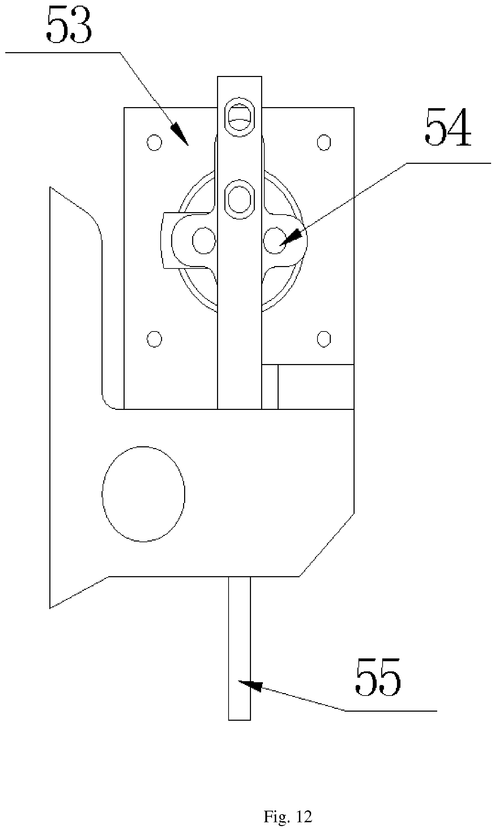

Further, as shown in Fig. 11 and Fig. 12, the downwards-pressing device 5 is used for downwards-pressing sample box, such that sample diluent in sample box can contact to test strip and carry out the reaction. The downwards-pressing device 5 comprises: a downwards-pressing head 55 is connected to the screw mandrel, as shown in Fig. 10, the downwards-pressing head 55 is connected to the third motor 53.

Further, as shown in Fig. 11 and Fig. 12, the downwards-pressing device 5 further comprises: a crank 54 is connected to the screw mandrel 22, the downwards-pressing head 55 is connected to the screw mandrel 22 by the crank 54. A rectangular hole 540 (also known as oblong hole) is provided in the crank 54, in other words, the downwards-pressing device 5 further comprises: a rotation shaft with oblong hole is connected the screw mandrel 22. The top of downwards-pressing head 55 is a connecting shaft, the connecting shaft is inserted into the rectangular hole 540 or movably provided in the rectangular hole 540. The rectangular hole 540 limits the top of downwards-pressing head 55 in vertical direction. When the crank 54 moving in circular movement, the downwards-pressing head 55 is limited in vertical direction so as to move up and down. By means of the motor driving the crank, the screw mandrel drives the crank in circular movement, such that the downwards-pressing head 55 is controllably driven in vertical direction so as to realize reciprocally up and down linear movement. Thus the downwards-pressing is finished in limited space, the structure of detection analyzer is more compact.

When the rectangular hole or oblong hole is under circular movement, the downwards-pressing head is realized reciprocally up and down linear movement in vertical direction. In starting status, the long axis of rectangular hole or oblong hole of crank parallels to the axial position of downwards-pressing head. When sensor C (namely sensor 24) detects the sample box entering into downwards-pressing area, the motor (third motor 53) drives the crank for rotation, the rectangular hole or oblong hole of crank rotates together with the crank, until the long axis of rectangular hole or oblong hole of crank is perpendicular to the axial position of downwards-pressing head, the downwards-pressing action is completed. Then the motor continues to rotate, the downwards-pressing components returns to starting status.

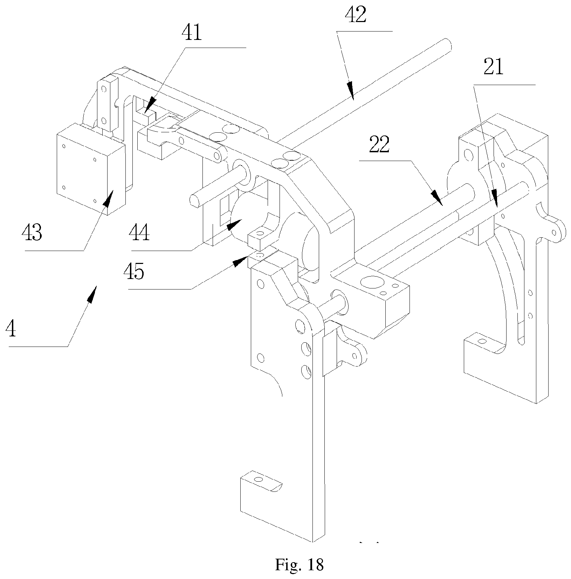

Further, the information collection device 4 is used for collecting the barcode information on sample box and taking a picture for detection result. As shown in Fig. 18, the information collection device 4 comprises: a barcode scanning device 43 and a camera 44, the barcode scanning device 43 scans label information on sample box (patient information on sample box), the camera 44 collects acquire color ribbon information presented on the test strips in the sample box.

The barcode scanning device 43 and camera 44 are connected to the screw mandrel 22. For example, the barcode scanning device 43 and camera 44 are connected to screw mandrel by feed screw nut on sample box frame mounting platform. When the sample boxes are lifted, the barcode scanning device 43 and camera 44 can synchronously scan the barcode and take pictures for sample boxes one by one along with movement of sample boxes or sample box frame. A row of sample boxes are lifted and removed together, then the sample boxes therefrom are downwards-pressed, detected, scanned and photographed one by one. Preparation works before downwards-pressing possess higher efficiency, and pertinence and correspondence of downwards-pressing, detecting, scanning and photographing are realized, thus efficiency and accuracy are realized.

When sample boxes are lifted to barcode scanning area, barcode scanning device and camera move perpendicular to linear track 21 in horizontal direction, and capture the information on sample boxes one by one. Barcode scanning sensor F (namely sensor 41) captures the signal, barcode scanning device begins to scan the barcode. Photographing sensor G (namely sensor 45) captures the signal, the camera photographs the detection result. The information collection device and downwards-pressing device use one electric motor or motor, thus the mechanism of detection analyzer is simplified.

All collected information is transmitted to information processing unit, the information processing unit processes the information according to preset program, the report of test result is stored in information processing unit. According to customer's requirement, test report can be printed or directly view in display screen or operating panel of detection analyzer.

A controlling unit controls all actions of detection analyzer, and controls coordinated operation of each assemblies by a controller, thus the detection analyzer finishes detection and analysis function. Controlling key of controlling unit can be provided on the operating panel. From pressing the sample box, sample diluent and test strip contacting, to photographing and collecting test result information, the time is accurately controlled at 5 minutes.

Firstly, the invention uses gudieways and sliding block for conveying the sample box, the sliding block is driven by motor, the movement of sliding block is more smooth and is controlled more accurate. Secondly, all sample boxes are located in the same sample box frame, batch feeding and batch recovery of sample boxes are realized. Thirdly, protrusion on sample box frame mounting platform works in with the groove of sample box frame, a sample box fixing lock is provided in the sample box frame, such that stability of sample box is ensured to the utmost during conveying the sample box. Thus device malfunctions due to the tilt of sample box can be effectively avoided. Fourthly, components and parts of this detection analyzer are simplified to the utmost, the analyzer is more compact and portable. Fifthly, the present invention mainly uses gear and screw mandrel for conveying, power conveying and movement controlling are more accurate.

The foregoing descriptions are merely specific schematic embodiments of present invention, and are by no means intended to limit the scope thereof. All components of present invention may be combined with each other if there is no conflict. Equivalent changes and modifications made by those skilled in the art without departing from the concept and principles of present invention should fall within the scope of protection of present invention.

## Claims

1. An array-type fecal occult blood detection analyzer, wherein the array-type fecal occult blood detection analyzer comprises:
a sample box frame for carrying multiple sample boxes, the sample box frame is an array-type structure;
a conveying unit for conveying the sample box frame, the conveying unit comprises: a linear track for supporting the sample box frame, and a driving device for driving the sample box frame so as to realize reciprocating linear movement;
a downwards-pressing device for downwards pressing the sample boxes; and
an information collection device for collecting color ribbon information presented on the test strip in the sample box and label information on the sample box.

2. The array-type fecal occult blood detection analyzer according to claim 1, whrerein the sample box frame is fixed in the sample box frame mounting platform, the sample box frame mounting platform is supported in the linear track by bearing;
the driving device is a first motor, the conveying unit further comprises: a screw mandrel is connected to the first motor, the sample box frame mounting platform is sleeve jointed to the screw mandrel by screw thread.

3. The array-type fecal occult blood detection analyzer according to claim 1, whrerein the sample box frame is rectangular matrix structure, multi row and multi column through holes are provided in the sample box frame for accommodating the sample boxes, elastic clamping groove is provided in the top of each sample box;
the array-type fecal occult blood detection analyzer further comprises: a sample box landing unit for raising all sample boxes in one horizontal rank of sample box frame;
the sample box landing unit comprises:
multiple bottle jacking-up rods are arranged in a row, the arrangement direction of bottle jacking-up rods and the horizontal direction of sample box frame are same, the number of bottle jacking-up rods and the number of horizontal sample boxes in the sample box frame, the bottle jacking-up rod can move up and down, and are provided below the through holes of sample box frame;
bottle lifting rod can move up and down along the horizontal direction of sample box frame, and are provided above the through holes of sample box frame, the length of bottle lifting rod is larger than the horizontal length of sample box frame, and the bottle lifting rod can extend into a row of elastic clamping grooves and is clamped by the elastic clamping grooves;
bottle removing rod can move up and down along the horizontal direction of sample box frame, and is provided above the through holes of sample box frame, the elastic clamping groove can be separated from the bottle lifting rod by downwards-pressing the bottle removing rod.

4. The array-type fecal occult blood detection analyzer according to claim 1, whrerein the array-type fecal occult blood detection analyzer further comprises: a locking device is used for locking the position of sample box frame, the locking device is connected to the second motor.

5. The array-type fecal occult blood detection analyzer according to claim 4, whrerein the locking device comprises: an eccentric shaft, a mandril flange is pressed below the eccentric shaft by the spring, and a mandril is connected to the mandril flange, the mandril flange is provided on the side of mandril, a locking groove is provided in the sample box frame, the mandril can reciprocally move up and down through the locking groove.

6. The array-type fecal occult blood detection analyzer according to claim 5, whrerein the locking device further comprises: a first sensor is used for detecting the rotation position of eccentric shaft, the first sensor is connected the second motor.

7. The array-type fecal occult blood detection analyzer according to claim 2, whrerein the sample box frame is fixed above the sample box frame mounting platform, and the sample box frame is clamped to the sample box frame mounting platform, a guiding rod is provided on the top surface of sample box frame mounting platform, a guiding groove is provided on the bottom of sample box frame, the guiding rod is positioned in the guiding groove, a second sensor is provided in the sample box frame mounting platform for detecting whether the sample box frame is fixed in place.

8. The array-type fecal occult blood detection analyzer according to claim 2, whrerein the downwards-pressing device comprises: a downwards-pressing head is connected to the screw mandrel, and the downwards-pressing head is connected to the third motor.

9. The array-type fecal occult blood detection analyzer according to claim 8, whrerein the downwards-pressing device further comprises: a crank is connected to the screw mandrel, the downwards-pressing head is connected to the screw mandrel by the crank, a rectangular hole is provided in the crank, the top of downwards-pressing head is connected into the rectangular hole, the rectangular hole limits the top of downwards-pressing head in vertical direction, when the crank moving in circular movement, the downwards-pressing head is limited in vertical direction so as to move up and down.

10. The array-type fecal occult blood detection analyzer according to claim 2, whrerein the information collection device comprises: a barcode scanning device and a camera, the barcode scanning device and the camera are connected to the screw mandrel.
